# EUROPEAN PATENT SPECIFICATION

(11) **EP 1 062 200 B1**
(45) Date of publication and mention of the grant of the patent: **10.11.2004**
(21) Application number: 99912720.2
(22) Date of filing: 19.03.1999
(51) Int. Cl.: C07C 253/30, C07C 255/24

(54) **AMINONITRILE PRODUCTION**
VERFAHREN ZUR HERSTELLUNG VON AMINONITRILEN
PRODUCTION D'AMINONITRILE

(30) Priority: 20.03.1998 US 78838 P; 20.03.1998 US 78715 P; 15.03.1999 US 268147; 15.03.1999 US 268148
(43) Date of publication of application: 27.12.2000
(60) Divisional application: 04075360.0
(73) Proprietor: INVISTA Technologies S.à.r.l., Wilmington, DE 19805 (US)
(72) Inventor: IONKIN, Alex, Sergey, Kennet Square, Pennsylvania 19348 (US); ZIEMECKI, Stanislaw, Bogdan, Wilmington, DE 19805 (US); HARPER, Mark, Jay, Lewis, DE 19958 (US); KOCH, Theodore, Augur, Wilmington, DE 19808 (US)
(74) Representative: Carpmaels & Ransford
(86) International application number: PCT/US1999/006044
(87) International publication number: WO 1999/047492

(56) References cited:
- EP-A- 0 077 911
- EP-A- 0 161 419
- WO-A-92/21650
- DE-A- 1 543 794
- DE-A- 19 636 768
- US-A- 3 591 618

## Description

### FIELD OF THE INVENTION

The invention relates to a selective hydrogenation process for producing aminonitriles.

### BACKGROUND OF THE INVENTION

Aminonitriles are a class of important chemicals which have a variety of industrial applications. For example, aminonitriles can be used as monomers for producing high molecular weight polyamides. Specifically, 6-aminocapronitrile can be used to produce nylon 6.

Aminonitriles can be produced by catalytic hydrogenation of dinitriles. However, the yield of and selectivity to a desired aminonitrile using processes known to one skilled in the art are generally not as high as one skilled in the art desires. Additionally, the amount of the complete hydrogenation product, diamine, is generally higher than one skilled in the art would desire.

A convenient process resulting in a high yield of aminonitriles at low levels of dinitrile starting materials, fully hydrogenated products (diamines) and byproducts would be of great usefulness for commercial production of aminonitriles.

Therefore, there is an increasing need to develop a process that can selectively hydrogenate a dinitrile to an aminonitrile.

DE-A-196 36 768 discloses a process for the selective hydrogenation of an aliphatic dinitrile to an aminonitrile by contacting the dinitrile with a hydrogen-containing fluid in the presence of a hydrogenation catalyst containing cobalt and iron and a solvent comprising liquid ammonia or an alcohol. The catalyst may be supported as by porous oxides such as magnesium oxide, and activated carbon.

WO-A-92/21650 discloses the selective hydrogenation of aliphatic dinitriles to aminonitriles using a Raney-type catalyst such as Raney nickel, and a solvent comprising (i) ammonia or (ii) a C₁ to C₄ alcohol containing a soluble inorganic base selected from alkali metal compounds and alkaline earth metal compounds.

### SUMMARY OF THE INVENTION

It is the object of the present invention to provide a process for the production of aminonitriles from dinitriles. An advantage of this invention is that an aminonitrile can be produced in high yield and having a high selectivity to the aminonitrile. Other objects and advantages will become more apparent as the invention is more fully disclosed hereinbelow.

This invention generally provides a process for the selective or partial hydrogenation of a dinitrile to an aminonitrile. The process comprises contacting a dinitrile with a hydrogen-containing fluid in the presence of (a) a solvent comprising liquid ammonia, an alcohol, or both; (b) a hydrogenation catalyst; and (c) an additive comprising a carbonyl group-containing organic compound.

### DETAILED DESCRIPTION OF THE INVENTION

According to this invention, a dinitrile having the formula of NCRCN is contacted with a hydrogen-containing fluid in the presence of a solvent, a hydrogenation catalyst, and an additive comprising a carbonyl group-containing organic compound selected from the group consisting of organic amides, organic esters, salts of carboxylic acids, and combinations of two or more thereof in which R is a hydrocarbylene group selected from the group consisting of alkylene group, arylene group, alkenylene group; alkarylene group, aralkylene group, and combinations of two or more thereof. The presently preferred R is an alkylene group. Each hydrocarbylene group can contain 2 to 25, preferably 2 to 15, and most preferably 2 to 10 carbon atoms per group. In other words, a suitable dinitrile can contain 4 to 27, preferably 4 to 17, and most preferably 4 to 12 carbon atoms per dinitrile molecule.

Examples of suitable dinitriles include, but are not limited to, adiponitrile, methylglutaronitrile, alpha, omega-propanedinitrile, alpha, omega-butanedinitrile, alpha, omega-pentanedinitrile, alpha, omega-heptanedinitrile, alpha, omega-nonanedinitrile, alpha, omega-dodecanedinitrile, alpha, omega-pentadecanedinitrile, alpha, omega-icosanedinitrile, alpha, omega-tetracosanedinitrile, 3-methylhexanedinitrile; 2-methyl-4-methyleneoctanedinitrile, and combinations of two or more thereof. The presently preferred dinitrile is adiponitrile because its selective hydrogenation product, 6-aminocapronitrile, is a well-known monomer for polymerization applications.

Any hydrogen-containing fluid can be used in the invention as long as there is sufficient hydrogen in the fluid to selectively hydrogenate a dinitrile to an aminonitrile. The term "fluid" refers to liquid, gas, or both. The hydrogen content in the fluid can range from 1 to 100%, preferably 50 to about 100%, and most preferably 90 to 100% by volume. The presently preferred hydrogen-containing fluid is a pure hydrogen gas.

The molar ratio of hydrogen (in the hydrogen-containing fluid) to dinitrile is not critical as long as sufficient hydrogen is present to produce the desired aminonitrile. Hydrogen is generally used in excess. Hydrogen pressures are generally in the range of 50 to 2000 psi (0.345 to 13.79 MPa), with 200 to 1000 psi (1.42 to 6.89 MPa) preferred.

Any solvent that comprises either liquid ammonia or an alcohol can be used in the invention. The concentration of liquid ammonia in the solvent can range from 20 to 100%, preferably 50 to 100%, and most preferably 80% to 100%, by weight of total solvent. A pure liquid ammonia is presently preferred. However, if an alcohol is also present in the solvent, the concentration of ammonia can be adjusted based on the quantity of alcohol used which is disclosed hereinbelow. The molar ratio of ammonia to dinitrile can generally be in the range of from 1:1 to 30:1, preferably 2:1 to 20:1.

Any alcohol that can facilitate the selected hydrogenation of a dinitrile to an aminonitrile can be used in this invention. The presently preferred alcohol has one to 10, preferably one to 4, carbon atoms per molecule. Examples of suitable alcohols include, but are not limited to, methanol, ethanol, propanol, isopropyl alcohol, butanol, isobutyl alcohol, pentanol, hexanol, heptanol, octanol, nonanol, decanol, and combinations of two or more thereof. The presently most preferred alcohol is methanol. The alcohol can generally be present in the solvent in the concentration of from 20 to 100%, preferably 30 to 99%, by weight.

The alcohol solvent can further comprise a base which is substantially soluble in the solvent. The term "substantially" refers to "more than trivial". The presently preferred base is an inorganic base. Examples of inorganic bases include, but are not limited to, alkali metal oxides, alkaline earth metal oxides, alkali metal hydroxides, alkaline earth metal hydroxides, partially neutralized acids in which one or more protons of the acids are replaced with ammonium ion, alkali metal ions, alkaline earth metal ions, or combinations of two or more thereof. Examples of suitable bases include, but are not limited to lithium hydroxide, sodium hydroxide, sodium oxide, potassium hydroxide, sodium bicarbonate, sodium carbonate, potassium bicarbonate, or combinations of two or more thereof The presently most preferred bases are ammonia, lithium hydroxide, and sodium hydroxide for they are readily available and inexpensive.

A base can be present in the solvent in any quantity so long as the quantity can facilitate the selective hydrogenation of a dinitrile to an amino nitrile. Generally, a base can be present in the solvent in the range of from 1 to 10 weight %, based on the total weight of the dinitrile.

The catalyst in the process is a hydrogenation catalyst. The presently preferred catalyst is selected from the group consisting of iron, cobalt, nickel, rhodium, magnesia-supported nickel-iron, and combinations of two or more. The catalyst may also contain one or more promoters, for example, chromium, molybdenum, and tungsten. The catalyst can also be in the form of an alloy such as, for example, Raney nickel, in the form of individual metal, or in the form of solid solution of two or more metals.

The catalytic metal can also be supported on an inorganic support such as alumina, magnesium oxide, and combinations thereof. The metal can be supported on an inorganic support by any means known to one skilled in the art such as, for example, impregnation, coprecipitation, ion exchange, and combinations of two or more thereof The presently preferred inorganic support is magnesium oxide.

The carbonyl group-containing organic additive effects a selectivity improvement. The term "improvement" is referred to as enhanced selectivity to aminonitrile product at conversions greater than 70%, preferably conversions greater than 80%, as compared to the selectivity without the use of the additive of this invention. A preferred carbonyl group-containing organic compound is selected from the group consisting of organic amides, organic esters, salts of carboxylic acids, urea, and combinations of two or more thereof.

Examples of suitable carbonyl-containing organic compounds include, but are not limited to, formamide, acetamide, N-methyl formamide, N,N-dimethyl formamide, N-methylacetamide, N-methyldodecanamide, methyl formate, ethyl formate, sodium formate, ammonium formate, urea, and combinations of two or more thereof.

The additive can be present during the contacting in any quantity which can improve the selective hydrogenation of a dinitrile to its corresponding aminonitrile. Generally, the weight ratio of the additive to the catalyst can be in the range of from 0.001:1 to 0.5:1, preferably 0.001:1 to 0.1:1.

The catalyst metal can be present in any physical shapes or forms. It can be in fluidizable forms, extrudates, tablets, spheres, or combinations of two or more thereof. The catalyst may be in sponge metal form, for example the Raney nickels and Raney cobalts. The molar ratio of a catalyst to dinitrile can be any ratio as long as the ratio can catalyze the selective hydrogenation of a dinitrile. The weight ratio generally can be in the range of from 0.0001:1 to 1:1, preferably 0.001:1 to 0.5:1. The process can be carried out in batch or continuous mode. If the catalytic metal is supported on an inorganic support or is a portion of alloy or solid solution, the catalytic metal can be present in the range of from 0.1 to 60, preferably 1 to 50, and most preferably 2 to 50 weight %, base on the total weight catalytic metal and inorganic support.

Catalyst and an additive disclosed above can be separately introduced into contacting with a dinitrile. However, it is presently preferred that a catalyst, whether it is in its metal form or in an alloy or solid solution or on an inorganic support, preferably in a solvent is contacted with an additive disclosed above. The solvent can be an alcohol, an ether, an ester, ammonia or combinations of two or more thereof Further preferably the contacting of a catalyst and an additive is also carried out in a hydrogen-containing fluid. The hydrogen-containing fluid can be the same as that disclosed above. Contacting of a catalyst and an additive produces a pretreated catalyst. The pretreated catalyst can be washed with a solvent disclosed above, preferably under anaerobic condition to produce an additive-treated catalyst.

The contacting of a catalyst and an additive can be carried out under any conditions effective to produce an additive-treated catalyst which can improve selective hydrogenation of a dinitrile or the selectivity to an aminonitrile. Generally, the entire process for producing the additive-treated catalyst can be carried out by contacting a catalyst with an additive disclosed above at a temperature in the range of from 20°C to 150°C, preferably 30°C to 100°C under a pressure in the range of from 0.5 to 100 atmospheres (atm) for 5 seconds to 25 hours.

Preferably the carbon atoms of the starting dinitrile are arranged in a branched or linear chain. Preferred examples are the hydrogenation of adiponitrile to 6-aminocapronitrile, methylglutaronitrile to two isomeric aminonitriles (5-amino-2-methylvaleronitrile and 5-amino-4-methylvaleronitrile), and alpha, omega-dodecanedinitrile to the corresponding aminonitrile. A most preferred application of the process of the present invention is to maximize the formation of 6-aminocapronitrile to simplify the production of nylon 6.

The process of the present invention can be carried out at a temperature in the range of from 25 to 150°C, preferably 40 to 100°C, most preferably 60 to 80°C at a total pressure from 50 to 2000 psi (0.345 to 13.79 Mpa), preferably 200 to 1000 psi (1.42 to 6.89 Mpa) for 15 minutes to 25 hours, preferably 1 hour to 10 hours.

The presently preferred catalyst is a sponge metal type catalyst. The metallic component is iron, cobalt or nickel. Commercially available catalysts of this type are promoted or unpromoted Raney Ni or Raney Co catalysts which can be obtained from the Grace Chemical Co. (Columbia, Maryland), Activated Metals Corporation (Sevierville, Tenn.) or Degussa (Ridgefield Park, NJ.). The metal catalyst, as disclosed above, can be supported on an inorganic support such as, for example, iron/nickel on magnesium oxide composition can be used as catalyst. In the case of the use of the supported nickel iron catalyst, the rate of adiponitrile conversion increases with the amount of Ni deposited on the support. The preferred concentration of Ni is between 5 and 50% (wt% of nickel in the metal/support composition), and especially between 25 and 35%. The iron present in the nickel/iron/ magnesium oxide composition can be between 0.2% and 20% of the Ni/Fe/MgO composition, most preferably 0.5% to 10%.

Wishing not to be bound by theory, it is believed that addition of an additive to, or use of additive-treated, catalyst in the selective hydrogenation process results in modification of the catalyst thereby improving or enhancing the selectivity to aminonitrile, compared to results obtained in the absence of such additives.

The process of the invention can be operated batch wise or continuously in an appropriate reactor. Stirring or agitation of the reaction mixture can be accomplished in a variety of ways known to those skilled in the art. The partial hydrogenation of the starting dinitrile to its corresponding aminonitrile with high selectivity at high conversions of the dinitrile makes this process efficient and useful.

The following examples further illustrate the process of the invention and are not to be construed to unduly limit the scope of the invention.

### EXAMPLES

### Definitions:

The meaning of terms used in the Examples is defined as follows.

Yield of aminonitrile is the measured concentration of aminonitrile divided by the starting concentration of dinitrile.

Conversion of the dinitrile is the difference between the starting and the instant concentration of dinitrile, divided by the starting concentration of dinitrile.

Selectivity to aminonitrile is the measured yield of aminonitrile divided by conversion of the dinitrile at that instance.

### Experiment 1 Preparation of NiFe/MgO catalysts

Distilled water (100 ml) was heated with stirring on a hot plate almost to the boiling point. The calculated amounts of nitrates of Fe(III) and Ni(II) were added together and at once. The nitrates dissolved after a few seconds of stirring. The solution was colored and had a pH of approximately 5. Magnesium oxide (Alfa Aesar, Ward Hill, Massachusetts) (3 g) was added slowly over several minutes to avoid boiling over of the solution. The heat was turned off, but vigorous stirring was continued for another 1-2 hours to allow for complete mixing and adsorption. The pH of the mixture at this point was about 11. After gradual cooling of the suspension, the reaction mixture was filtered and the solids were washed with distilled water. The washed solids were dried overnight in a vacuum oven (0.1 mm Hg) set at 115°C -120°C. The dried solid was ground with a mortar and pestle and was hereinafter referred to as ground catalyst or catalyst precursor.

The ground catalyst precursor was generally activated by heating in a tube furnace, in a flow of hydrogen of approximately 40 cc/min. The temperature was slowly raised to 480°C, then kept at that level overnight (ca. 14 hours). The reduced catalyst was cooled in a flow of hydrogen, then unloaded and stored in a dry box.

### Examples 1- 6

### Hydrogenation of Adiponitrile to Aminocapronitrile with NiFe/MgO Catalyst

Catalyst containing 3 5% of nickel and 2.5% of iron by weight was prepared and activated according to the method of Experiment 1. The activated catalyst was kept in a dry box.

The hydrogenation runs were carried out as follows. A portion of the activated catalyst (1.2 g or 0.6 g) was loaded into a 50 cc autoclave, then 3.2 g of adiponitrile containing a N-methyl pyrrolidinone internal standard was added, followed by (1) 35 ml (21 g) of liquid ammonia (Example 1) or (2) 3 5 ml of methanol and 0.5 g of 50 weight % NaOH (Examples 2-6) as solvent and hydrogen. The autoclave was operated at 70°C and 6.89 Mpa (1000 psig total)(hydrogen + ammonia) pressure, with liquid samples being withdrawn for analysis via a filter. The impurities detected were: about 60 ppm ofhexamethylene imine, 0.67% of 2-cyclopentylidene imine, 0.43% of cyanovaleroamide, and ca. 0.5% bishexamethylenetriamine.

Pertinent results of the above runs are summarized in the following Table I. As shown in the table, the invention runs produced aminocapronitrile at high selectivity to aminocapronitrile.

**Table I**

| Example No. | Catalyst (g) | Time (minute) | Yield (%) | Selectivity (%) | ADN Conversion |
|---|---|---|---|---|---|
| 1 | 35%Ni2.5%Fe/MgO (1.2) | 72 | 71.3 | 79.6 | 89% |
| 2 | 25%Ni5%Fe/MgO (0.6) | 90 | 71 | 80 | 90 |
| 3 | 35%Ni5%Fe/MgO (0.6) | 120 | 75 | 81.5 | 92 |
| 4 | 35%Ni/MgO (0.6) | 80 | 72 | 76 | 94 |
| 5 | 15%Ni/MgO (0.6) | 240 | 75 | 80 | 93 |
| 6 | 25%Ni/MgO (0.6) | 120 | 75.4 | 82 | 92 |

### Comparative Example 1

Raney Ni (1.2 g) promoted with Fe and Cr (Activated Metals, A4000, without any further additives) was added to a 50 cc autoclave together with 3.2 g adiponitrile (ADN) and 35cc of liquid ammonia to form a mixture. Hydrogen was introduced to the autoclave and the ADN was hydrogenated at 60°C under the total pressure of 7.2 MPa (1045 psig) at ca. 1500 rpm. Total conversion of ADN was reached within 30 minutes on stream. The maximum yield of aminocapronitrile reached 57%, for selectivity of 71% at 80% ADN conversion, and 63% at 90% ADN conversion.

### Comparative Example 2

To a 300 cc autoclave, was charged 7.7g Raney Co (obtained from W.R. Grace Co.), 0.77 g water, 26 g ADN, and 139 g liquid ammonia. The content was hydrogenated at 70°C, under the total pressure of 6.89 Mpa (1000 psig), at 1000 rpm. Total conversion of ADN was reached within 40 minutes on stream. The maximum yield of aminocapronitrile reached 58%, for selectivity of ca 74% at 80% ADN conversion, and ca 64% at 90% ADN conversion.

### Example 7-19

The runs were carried out as described above in Examples 1-6 with the exception that (1) some of the catalysts used in the runs were nickel, cobalt, or both, and some were promoted with chromium, (2) the catalysts used in these runs also comprised an additive noted in Table II, (3) the catalyst used in Example 17 was a nickel/cobalt catalyst promoted with chromium, and (4) the conditions specifically noted in the table.

In Example 17, a sponge catalyst comprising nickel and cobalt promoted with chromium was prepared, by leaching, from the alloy containing initially 54% Al, 23% Ni, 23% Co, and 1% Cr by weight. This catalyst was pretreated with formamide as described in below.

Pretreatment of catalyst with an additive was carried out as follows. Raney Ni (3.6 g) was charged into a 50 ml autoclave, together with 4.5 g of an additive shown in Table II. Subsequently 35 ml of liquid ammonia was added, and the mixture was heated to 80°C with stirring; the pressure was adjusted to 6.89 MPa to (1000 psig) with hydrogen, then kept under such conditions for 2.5 hours. After cooling the pressure was released, the sample was transferred to a dry box, washed with deaerated methanol, and stored under anaerobic conditions.

### Comparative Example 3

The catalyst described in the Example 17, but not pretreated, was tested in the ADN hydrogenation reaction in NH₄OH solvent. A 100 ml autoclave was charged with 1 g of the activated catalyst, 10 g of ADN mixed with 5 g MeOH and containing the internal standard, and 26.2 g of the 30% NH₄OH, pressurized with hydrogen to 3.45 MPa (500 psig), and run at 75°C. The maximum yield of aminocapronitrile was 58% at 76 % ADN conversion, thus selectivity of 76%.

The results of these runs are shown in the following Table II. Table II shows that the invention runs produced aminocapronitrile at substantially higher selectivity to aminocapronitrile than the results shown in the comparative examples.

**Table II**

| Example | Catalyst | Additive | Time, hour | Sel., % | Conv., % |
|---|---|---|---|---|---|
| comp. 1 | Raney Ni | none | 0.5 | 71 | 80 |
| comp. 2 | Raney Co | none | 0.7 | 74 | 80 |
| 7 | Raney Ni | formamide | 2 | 88 | 80 |
| 8 | Raney Ni^{a} | formamide | 1.7 | 93 | 80 |
| 9 | Raney Co^{a} | formamide | 3.5 | 84 | 80 |
| 10 | Raney Co | formamide | 0.7 | 78 | 80 |
| 11 | Raney Ni | N-meth. formamide^{e} | 1.7 | 88 | 80 |
| 12 | Raney Ni^{b} | N-meth. formamide^{e} | 0.7 | 86 | 80 |
| 13 | Raney Ni^{b} | amm. formate^{f} | 2 | 81 | 80 |
| 14 | Raney Ni^{a} | acetamide | 3.5 | 79 | 80 |
| 15 | Raney Ni^{a,b} | ethyl formate | 0.7 | 88 | 80 |
| 16 | Raney Fe/CoNi^{a} | formamide | 1.7 | 77 | 85 |
| 17 | Raney NiCo/Cr^{a} | formamide | 2.8 | 76 | 87 |
| comp. 3 | Raney NiCo/Cr^{c} | none | 1.5 | 76 | 76 |
| 18 | Raney Ni^{a,d} | urea | 1 | 84 | 85 |
| 19 | Raney Co^{a,b} | ethyl formate | 2.5 | 87 | 77 |
| Footnote: All hydrogenation experiments run at 6.89 MPa (1000 psig) total, at 70°C unless otherwise indicated. a) catalyst pretreated with an additive; b) run at 80°C; c) at 75°C; d) at 60°C; e) N-methyl formamide; and f) ammonia formate. | | | | | |

## Claims

1. A process for the selective hydrogenation of a dinitrile to an aminonitrile by contacting the dinitrile with a hydrogen-containing fluid in the presence of a hydrogenation catalyst and a solvent comprising liquid ammonia, an alcohol or both, **characterized in that** the components are contacted in the further presence of an additive comprising a carbonyl group-containing organic compound.

2. The process according to claim 1, **characterized in that** the dinitrile has the formula NCRCN, wherein R is an alkylene, arylene, alkenylene, alkarylene or aralkylene group having from 2 to 25 carbon atoms.

3. The process according to claim 2, **characterized in that** the dintrite is adiponitrile.

4. The process according to claim 1, **characterized in that** the hydrogenation catalyst comprises a metal selected from the group consisting of iron, cobalt, nickel, rhodium and combinations of two or more thereof

5. The process according to claim 1, **characterized in that** the hydrogenation catalyst is supported on an inorganic support.

6. The process according to claim 1, **characterized in that** the hydrogenation catalyst and additive are contacted to produce an additive-treated catalyst before being contacted with the dinitrile.

7. The process according to claim 1, **characterized in that** the catalyst and additive are used in a weight ratio of from 0.0001:1 to 1:1

8. The process according to claim 1, **characterized in that** the components are contacted at a temperature of from 25 to 150°C and a total pressure of from 0.345 to 13.79 MPa, for from 15 minutes to 25 hours.

9. The process according to any one of claim 1 to 8, **characterized in that** the carbonyl group-containing organic compound is selected from the group consisting of organic amides, organic esters, carboxylic acids, salts of carboxylic acids, urea and combinations of two or more thereof.

10. The process according to claim 9, **characterized in that** the additive is an amide.

11. The process according to claim 9, **characterized in that** the additive is a carboxylic acid salt.

12. The process according to claim 9, **characterized in that** the additive is an ester.

13. The process according to claim 9, **characterized in that** the additive is urea.

14. The process according to claim 9, **characterized in that** the additive is selected from the group consisting of formamide, acetamide, N-methyl formamide, N,N-dimethyl formamide, N-methylacetamide, N-methyldodecanamide, methyl formate, ethyl formate, sodium formate, ammonium formate, urea and combinations of two or more thereof.

## Patentansprüche

1. Verfahren für die selektive Hydrierung eines Dinitrils zu einem Aminonitril durch Inkontaktbringen des Dinitrils mit einem wasserstoffhaltigen Fluid in Anwesenheit eines Hydrierungskatalysators und eines Lösungsmittels, umfassend flüssigen Ammoniak, einen Alkohol oder beide, **dadurch gekennzeichnet, daß** die Komponenten in der weiteren Anwesenheit eines Zusatzstoffes, umfassend eine carbonylgruppenhaltige organische Verbindung, in Kontakt gebracht werden.

2. Verfahren nach Anspruch 1, **dadurch gekennzeichnet, daß** das Dinitril die Formel NCRCN hat, wobei R eine Alkylen-, Arylen-, Alkenylen-, Alkarylen- oder Aralkylengruppe mit 2 bis 25 Kohlenstoffatomen ist.

3. Verfahren nach Anspruch 2, **dadurch gekennzeichnet, daß** das Dinitril Adiponitril ist.

4. Verfahren nach Anspruch 1, **dadurch gekennzeichnet, daß** der Hydrierungskatalysator ein Metall, ausgewählt aus der Gruppe, bestehend aus Eisen, Cobalt, Nickel, Rhodium und Kombinationen von zwei oder mehreren davon, umfaßt.

5. Verfahren nach Anspruch 1, **dadurch gekennzeichnet, daß** der Hydrierungskatalysator auf einem anorganischen Träger geträgert ist.

6. Verfahren nach Anspruch 1, **dadurch gekennzeichnet, daß** der Hydrierungskatalysator und der Zusatzstoff in Kontakt gebracht werden, um einen mit Zusatzstoff behandelten Katalysator zu erzeugen, bevor sie mit dem Dinitril in Kontakt gebracht werden.

7. Verfahren nach Anspruch 1, **dadurch gekennzeichnet, daß** der Katalysator und der Zusatzstoff in einem Gewichtsverhältnis von 0,0001:1 bis 1: 1 verwendet werden.

8. Verfahren nach Anspruch 1, **dadurch gekennzeichnet, daß** die Komponenten bei einer Temperatur von 25 bis 150°C und einem Gesamtdruck von 0,345 bis 13,79 MPa für 15 Minuten bis 25 Stunden in Kontakt gebracht werden.

9. Verfahren nach einem der Ansprüche 1 bis 8, **dadurch gekennzeichnet, daß** die carbonylgruppenhaltige organische Verbindung aus der Gruppe, bestehend aus organischen Amiden, organischen Estern, Carbonsäuren, Salzen von Carbonsäuren, Harnstoff und Kombinationen von zwei oder mehreren davon, ausgewählt ist.

10. Verfahren nach Anspruch 9, **dadurch gekennzeichnet, daß** der Zusatzstoff ein Amid ist.

11. Verfahren nach Anspruch 9, **dadurch gekennzeichnet, daß** der Zusatzstoff ein Carbonsäuresalz ist.

12. Verfahren nach Anspruch 9, **dadurch gekennzeichnet, daß** der Zusatzstoff ein Ester ist.

13. Verfahren nach Anspruch 9, **dadurch gekennzeichnet, daß** der Zusatzstoff Harnstoff ist.

14. Verfahren nach Anspruch 9, **dadurch gekennzeichnet, daß** der Zusatzstoff aus der Gruppe, bestehend aus Formamid, Acetamid, N-Methylformamid, N,N-Dimethylformamid, N-Methylacetamid, N-Methyldodecanamid, Methylformiat, Ethylformiat, Natriumformiat, Ammoniumformiat, Harnstoff und Kombinationen von zwei oder mehreren davon, ausgewählt ist.

## Revendications

1. Procédé pour l'hydrogénation sélective d'un dinitrile en un aminonitrile par mise en contact du dinitrile avec un fluide contenant de l'hydrogène en présence d'un catalyseur d'hydrogénation et d'un solvant comprenant de l'ammoniaque liquide, un alcool ou les deux, **caractérisé en ce que** les composants sont mis en contact en la présence supplémentaire d'un additif comprenant un composé organique contenant un groupe carbonyle.

2. Procédé selon la revendication 1, **caractérisé en ce que** le dinitrile a la formule NCRCN, dans laquelle R représente un groupe alkylène, arylène, alcénylène, alcarylène ou aralkylène ayant de 2 à 25 atomes de carbone.

3. Procédé selon la revendication 2, **caractérisé en ce que** le dinitrile est de l'adiponitrile.

4. Procédé selon la revendication 1, **caractérisé en ce que** le catalyseur d'hydrogénation comprend un métal choisi parmi le groupe constitué du fer, du cobalt, du nickel, du rhodium et de combinaisons de deux ou plusieurs d'entre eux.

5. Procédé selon la revendication 1, **caractérisé en ce que** le catalyseur d'hydrogénation est supporté sur un support inorganique.

6. Procédé selon la revendication 1, **caractérisé en ce que** le catalyseur d'hydrogénation et l'additif sont mis en contact pour produire un catalyseur traité par un additif avant d'être mis en contact avec le dinitrile.

7. Procédé selon la revendication 1, **caractérisé en ce que** le catalyseur et l'additif sont utilisés en un rapport de poids de 0,0001: 1 à 1:1.

8. Procédé selon la revendication 1, **caractérisé en ce que** les composants sont mis en contact à une température allant de 25 à 150°C et une pression totale allant de 0,345 à 13,79 MPa, durant une période allant de 15 minutes à 25 heures.

9. Procédé selon l'une quelconque des revendications 1 à 8, **caractérisé en ce que** le composé organique contenant un groupe carbonyle est choisi parmi le groupe constitué des amides organiques, des esters organiques, des acides carboxyliques, des sels d'acides carboxyliques, de l'urée et de combinaisons de deux ou plusieurs d'entre eux.

10. Procédé selon la revendication 9, **caractérisé en ce que** l'additif est un amide.

11. Procédé selon la revendication 9, **caractérisé en ce que** l'additif est un sel d'acide carboxylique.

12. Procédé selon la revendication 9, **caractérisé en ce que** l'additif est un ester.

13. Procédé selon la revendication 9, **caractérisé en ce que** l'additif est de l'urée.

14. Procédé selon la revendication 9, **caractérisé en ce que** l'additif est choisi parmi le groupe constitué du formamide, de l'acétamide, du *N*-méthylformamide, du *N,N* diméthylformamide, du *N*-méthylacétamide, du *N*-méthyldodécanamide, du formiate de méthyle, du formiate d'éthyle, du formiate de sodium, du formiate d'ammonium, de l'urée et de combinaisons de deux ou plusieurs d'entre eux.
